# EUROPÄISCHE PATENTSCHRIFT

(11) **EP 2 552 880 B1**
(45) Veröffentlichungstag und Bekanntmachung des Hinweises auf die Patenterteilung: **07.03.2018**
(21) Anmeldenummer: 11710524.7
(22) Anmeldetag: 29.03.2011
(51) Int. Cl.: C07C 45/67, C07C 47/565, C07C 47/575, C07C 47/58

(54) **VERFAHREN ZUR HERSTELLUNG HYDROXY-SUBSTITUIERTER AROMATISCHER ALDEHYDE**
PROCESS FOR PREPARING HYDROXY-SUBSTITUTED AROMATIC ALDEHYDES
PROCÉDÉ DE PRODUCTION D'ALDÉHYDES AROMATIQUES À SUBSTITUTION HYDROXY

(30) Priorität: 01.04.2010 EP 10158910
(43) Veröffentlichungstag der Anmeldung: 06.02.2013
(73) Patentinhaber: BASF SE, 67056 Ludwigshafen am Rhein (DE)
(72) Erfinder: EBEL, Klaus, 68623 Lampertheim (DE); RÜDENAUER, Stefan, 67549 Worms (DE)
(74) Vertreter: BASF IP Association
(86) Internationale Anmeldenummer: PCT/EP2011/054851
(87) Internationale Veröffentlichungsnummer: WO 2011/120980

(56) Entgegenhaltungen:
- REN X ET AL: "First enantioselective synthesis of daphneticin and its regioisomer", TETRAHEDRON ASYMMETRY, PERGAMON PRESS LTD, OXFORD, GB, Bd. 13, Nr. 16, 27. August 2002 (2002-08-27), Seiten 1799-1804, XP004380812, ISSN: 0957-4166, DOI: DOI:10.1016/S0957-4166(02)00482-2 in der Anmeldung erwähnt
- BAO K. ET AL.: "Selective demethylation and debenzylation of aryl ethers by magnesium iodide under solvent-free conditions and its application to the total synthesis of natural products", ORGANIC & BIOMOLECULAR CHEMISTRY, Bd. 7, 2009, Seiten 5084-5090, XP002640085,
- H W Stewart ET AL: "Correlation of the Base Strengths of Amines'", J . Chem. Sac.c). Chem, 1 January 1957 (1957-01-01), page 1878, XP55343116, Retrieved from the Internet: URL:http://pubs.acs.org/doi/pdf/10.1021/ja 01577a030 [retrieved on 2017-02-07]

## Beschreibung

Gegenstand der Erfindung ist ein Verfahren zur Herstellung aromatischer Aldehyde, die über mindestens eine Hydroxygruppe am Aromaten verfügen.

Eine literatur-bekannte Verfahrensweise zur Herstellung Hydroxy-substituierter aromatischer Aldehyde ist z.B. die O-Demethylierung aromatischer Methylether. Die grosstechnische Nutzung bekannter Methoden zur O-Demethylierung ist jedoch begrenzt, da beispielsweise die eingesetzten Reagenzien kostspielig sind, die Korrosion fördern, und/oder sehr drastische Reaktionsbedingungen erfordern, wie z.B. die Umsetzung mittels Natriumthioethanolat bei 150°C in Dimethylformamid oder die Umsetzung mit Natrium in flüssigem Ammoniak. Umsetzungen mit hochreaktiven Reagenzien wie z.B. Bortribromid, Aluminiumtrichlorid oder Bortrichlorid führen zwar zu einem schnellen Umsatz, zeigen jedoch eine deutlich verminderte Selektivität, sobald zwei oder drei aromatische Methoxygruppen am Aromaten vorhanden sind. Der Einsatz starker Lewis-Säuren oder Lewis-Basen ist auch dann erschwert, wenn der Aromat weitere funktionelle Gruppen enthält, wie z.B. Aldehyd-, Keto- oder benzylische AlkoholGruppen, da bei dieser Konstellation häufiger Nebenreaktionen auftreten können.

Die regioselektive O-Demethylierung mehrfach Methoxy-substituierter Benzaldehyde ist aus der Literatur bekannt, wie z.B.:
Demyttenaere et al., Tetrahedron 2002, 58, 2163 - 2166, verwendet AlCl₃ zur Demethylierung von 2,3,4-Trimethoxybenzaldehyd. Die Demethylierung erfolgt in Gegenwart eines grossen Überschusses von AlCl₃ in ortho- und para-Position.
Ren et al., Tetrahedron Asymm. 2002, 13, 1799 - 1804, verwendet eine Piperidin-Wasser-Mischung zur Demethylierung von 2,3,4-Trimethoxybenzaldehyd. Nachteilig sind die langen Reaktionszeiten und die leicht reduzierte Anwendbarkeit dieser Umsetzung auf nur stark elektronenreiche Verbindungen.
Bhattacharya et al. Tetrahedron Lett. 2006, 565 - 567, verwendet NaSCN und Triton-X 405 zur Demethylierung von 3,4-Dimethoxybenzaldehyd. Diese Umsetzung erfordert relativ hohe Reaktionstemperaturen.
Fang et al., J. Mol. Cat.A: Chemical 2007, 16 - 23, verwendet LiCl in Dimethylformamid unter Mikrowelleneinstrahlung zur Demethylierung von 2,3,4-Trimethoxybenzaldehyd und 3,4-Dimethoxybenzaldehyd. Die Umsetzung wird in Gegenwart von kostspieligem LiCl durchgeführt. Bei der Umsetzung bildet sich ferner toxisches Methylchlorid.
Prager und Tan, Tetrahedron Lett. 1967, 38, 3661-3664, verwendet Lewis-Säuren, wie z.B. AlCl₃ zur Demethylierung von 3,4-Dimethoxybenzaldehyd. Nachteilig bei dieser Verfahrensweise sind die Korrosionsprobleme dieses Reagenzes sowie die erforderliche exakte Einstellung vom Molverhältnis.
Pearl et al., J. Am. Chem. Soc. 1952, 74, 4262 - 4263, verwendet Schwefelsäure zur Demethylierung von 2,3,4-Trimethoxybenzaldehyd. Nachteilig sind die Korrosionsprobleme dieses Reagenzes, sowie bei der Anwendung dieser Verfahrensweise auf 3,4-Dimethoxybenzaldehyd im Vergleich zum 2,3,4-Trimethoxybenzaldehyd eine Umsetzung in umgekehrter Regioselektivität.

Ausgehend von diesem Stand der Technik bestand die Aufgabe der vorliegenden Erfindung darin, ein Verfahren zur Herstellung eines Hydroxy-substituierten aromatischen Aldehyds bereitzustellen, das sich auf verfahrenstechnisch gut handhabbare Weise und mit hoher Gesamtausbeute bei möglichst hoher Regioselektivität im technischen Maßstab durchführen lässt. Dabei sollen wohlfeile, leicht wiederzugewinnende und gut wieder einsetzbare Ausgangsverbindungen und Reagenzien genutzt werden können.

Überraschenderweise wurde gefunden, dass die Umsetzung von Alkoxybenzaldehyden mit Dialkylamin bei erhöhter Temperatur und erhöhtem Druck zu einer schnellen und auch regioselekiven O-Dealkylierung führt.

Gegenstand der vorliegenden Erfindung ist somit ein Verfahren zur Herstellung eines Aldehyds der Formel (I) 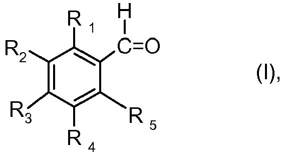 wobei einer, zwei oder alle drei Reste der Gruppe R₁, R₃ und R₅ Hydroxy bedeuten, und derjenige Rest oder diejenigen Reste der Gruppe R₁, R₃ und R₅ die nicht Hydroxy bedeuten, unabhängig voneinander Wasserstoff, C₁-C₈-Alkyl oder C₆-C₁₄-Aryl sind, und R₂ und R₄ unabhängig voneinander Wasserstoff, C₁-C₈-Alkyl, C₁-C₈-Alkoxy oder C₆-C₁₄-Aryl bedeuten, welches dadurch gekennzeichnet ist, dass man einen Aldehyd der Formel (II) 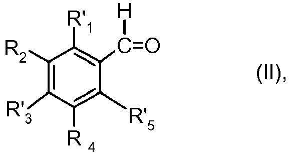 worin einer, zwei oder alle drei Reste der Gruppe R'₁, R'₃ und R'₅ C₁-C₈-Alkoxy bedeuten, und derjenige Rest oder diejenigen Reste der Gruppe R'₁, R'₃ und R'₅ die nicht C₁-C₈-Alkoxy bedeuten, unabhängig voneinander Wasserstoff, C₁-C₈-Alkyl oder C₆-C₁₄-Aryl bedeuten, und R₂ und R₄ die unter Formel (I) angegebenen Bedeutungen haben, bei einer Temperatur im Bereich zwischen 40°C und 300 °C und unter Druck zwischen 1 und 100 bar in Gegenwart von Dimethylamin oder Diethylamin umsetzt, und anschliessend das Reaktionsprodukt der Formel (I) isoliert.

Das erfindungsgemäße Verfahren zeichnet sich durch eine überraschend hohe Regioselektivität aus. So wird z.B. nach dem erfindungsgemäßen Verfahren ein 3,4,5-Trimethoxybenzaldehyd sehr selektiv zu dem 4-Hydroxy-3,5-dimethoxybenzaldehyd umgesetzt.

Für die Reste R₁, R₂, R₃, R₄, R₅, R'₁, R'₃ und R'₅ als C₁-C₈-Alkyl kommen z.B. in Betracht: Methyl, Ethyl, n-Propyl, Isopropyl, n-Butyl, sec-Butyl, tert-Butyl, Isobutyl, n-Pentyl, n-Hexyl oder n-Octyl, wobei die genannten C₁-C₈-Alkylreste substituiert sein können, wie z.B. durch Halogen, wie Chlor, Fluor oder Brom, oder Hydroxy. Bevorzugt sind C₁-C₄-Alkylreste und besonders bevorzugt C₁-C₃-Alkylreste. Ganz besonders bevorzugte Alkylreste sind Methyl und Ethyl.

Für die Reste R₁, R₂, R₃, R₄, R₅, R'₁, R'₃ und R'₅ als C₆-C₁₄-Aryl kommen vorzugsweise der Phenyl- oder Naphthylrest in Betracht, wobei diese Reste am aromatischen Kern weitersubstituiert sein können, wie z.B. durch Halogen, wie z.B. Fluor, Chlor oder Brom, C₁-C₄-Alkyl, wie z.B. Methyl, Ethyl, n-Propyl, Isopropyl, n-Butyl, sec-Butyl, tert-Butyl und Isobutyl, C₁-C₄-Alkoxy, wie z. B. Methoxy, Ethoxy, n-Propoxy, Isopropoxy, n-Butoxy und tert-Butoxy.

Für die Reste R₂, R₄, R'₁, R'₃ und R'₅ als C₁-C₈-Alkoxy kommen z.B. in Betracht: Methoxy, Ethoxy, n-Propoxy, Isopropoxy, n-Butoxy, tert-Butoxy, n- Pentyloxy und n-Octyloxy. Bevorzugt sind C₁-C₄-Alkoxyreste und besonders bevorzugte Reste sind Methoxy und Ethoxy. Für die Reste R'₁, R'₃ und R'₅ ist der Methoxyrest ganz besonders bevorzugt. Für die Reste R₂ und R₄ kommen als C₁-C₄-Alkoxyrest ganz besonders bevorzugt Methoxy und Ethoxy in Betracht.

Die erfindungsgemäße Umsetzung erfolgt bei einer Temperatur im Bereich zwischen 40 und 300°C, besonders bevorzugt im Bereich zwischen 60 und 250°C, und ganz besonders bevorzugt im Bereich zwischen 80 und 160°C.

Das erfindungsgemäße Verfahren wird unter Druck zwischen 1 und 100 bar ausgeführt, wobei ein Druck zwischen 2 und 60 bar bevorzugt ist. Ganz besonders bevorzugt ist ein Druck zwischen 2 und 20 bar.

Die Reaktion kann über einen längeren Zeitraum ausgeführt werden, wobei die Reaktionsdauer der Umsetzung im Bereich zwischen 1 und 48 Stunden liegt. Bevorzugt ist eine Reaktionsdauer zwischen 2 und 18 Stunden, und ganz besonders bevorzugt liegt die Reaktionsdauer zwischen 6 und 15 Stunden.

Das erfindungsgemäße Verfahren wird in Gegenwart von Dialkyaminen ausgewählt aus der Gruppe bestehend aus Dimethylamin und Diethylamin ausgeführt Besonders bevorzugt ist Dimethylamin.

Eine ebenfalls bevorzugte Ausgestaltung des erfindungsgemäßen Verfahrens ist dadurch gekennzeichnet, dass die Umsetzung in Gegenwart des Dialkylamins in wässriger Lösung erfolgt. Dimethylamin ist sehr gut wasserlöslich. Es können somit handelsübliche wässrige Lösungen von Dimethylamin in Wasser eingesetzt werden. Handelsübliche Lösungen von Dimethylamin in Wasser enthalten ca. 35 bis 65 Mol-% Dimethylamin. Diethylamin ist flüssig und kann ebenfalls als wässrige Lösung eingesetzt werden. In einer bevorzugten Ausführungsform des erfindungsgemäßen Verfahrens wird Dimethylamin in wässriger Lösung eingesetzt. Der Gehalt der wässrigen Lösung an Dimethylamin beträgt vorzugsweise 40 bis 60 Mol-%.

In einer weiteren Ausführungsform kann das erfindungsgemäße Verfahren in Gegenwart eines oder mehrerer weiterer Lösungsmittel ausgeführt werden. Als weitere Lösungsmittel kommen insbesondere polare Lösungsmittel, wie z.B. Acetonitril, Aceton, Ethylacetat oder auch Alkohole wie z.B. Methanol, Ethanol in Betracht.

Eine weitere bevorzugte Ausführungsform des erfindungsgemäßen Verfahrens ist dadurch gekennzeichnet, dass ein Aldehyd der Formel (II) eingesetzt, worin einer, zwei oder alle drei Rest der Gruppe R'₁, R'₃ und R'₅ C₁-C₄-Alkoxy, insbesondere Methoxy, bedeuten, und derjenige Rest oder diejenigen Reste der Gruppe R'₁, R'₃ und R'₅ die nicht C₁-C₄-Alkoxy, insbesondere Methoxy, sind, unabhängig voneinander Wasserstoff, C₁-C₄-Alkyl oder C₆-C₁₀-Aryl bedeuten, und R₂ und R₄ unabhängig voneinander Wasserstoff, C₁-C₈-Alkyl, C₁-C₈-Alkoxy oder C₆-C₁₄-Aryl bedeuten.

Eine besonders bevorzugte Ausführungsform des erfindungsgemäßen Verfahrens ist dadurch gekennzeichnet, dass man einen Aldehyd der Formel (II) einsetzt, worin einer, zwei oder alle drei Reste der Gruppe R'₁, R'₃ und R'₅ Methoxy bedeuten, und derjenige Rest oder diejenigen Reste der Gruppe R'₁, R'₃ und R'₅ die nicht Methoxy bedeuten, unabhängig voneinander Wasserstoff, C₁-C₄-Alkyl oder C₆-C₁₀-Aryl bedeuten, und R₂ und R₄ Wasserstoff, C₁-C₄-Alkyl, C₁-C₄-Alkoxy oder C₆-C₁₀-Aryl bedeuten.

Eine ganz besonders bevorzugte Ausführungsform des erfindungsgemäßen Verfahrens ist dadurch gekennzeichnet, dass man einen Aldehyd der Formel (IIa), 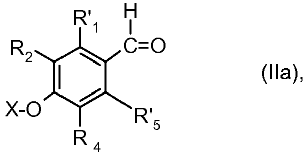 worin R'₁ und R'₅ unabhängig voneinander Wasserstoff, C₁-C₄-Alkyl oder C₆-C₁₀-Aryl bedeuten, und R₂ und R₄ unabhängig voneinander Wasserstoff, C₁-C₄-Alkyl, C₁-C₄-Alkoxy oder C₆-C₁₀-Aryl bedeuten, und X C₁-C₄-Alkyl, insbesondere Methyl, ist, zu einem Aldehyd der Formel (la) 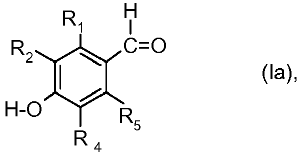 umsetzt, worin R₁ und R₅ unabhängig voneinander Wasserstoff, C₁-C₄-Alkyl oder C₆-C₁₀-Aryl bedeuten, und R₂ und R₄ unabhängig voneinander Wasserstoff, C₁-C₄-Alkyl, C₁-C₄-Alkoxy oder C₆-C₁₀-Aryl bedeuten.

Eine ebenfalls ganz besonders bevorzugte Ausführungsform des erfindungsgemäßen Verfahrens ist dadurch gekennzeichnet, dass man einen Aldehyd der Formel (IIb), 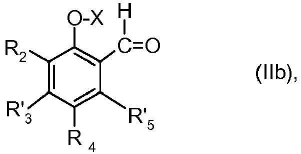 worin R'₃ und R'₅ unabhängig voneinander Wasserstoff, C₁-C₄-Alkyl oder C₆-C₁₀-Aryl bedeuten, und R₂ und R₄ unabhängig voneinander Wasserstoff, C₁-C₄-Alkyl, C₁-C₄-Alkoxy oder C₆-C₁₀-Aryl bedeuten, und X C₁-C₄-Alkyl, insbesondere Methyl ist, zu einem Aldehyd der Formel (Ib) 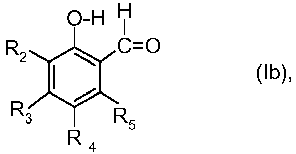 umsetzt, worin R₃ und R₅ unabhängig voneinander Wasserstoff, C₁-C₄-Alkyl oder C₆-C₁₀-Aryl bedeuten, und R₂ und R₄ unabhängig voneinander Wasserstoff, C₁-C₄-Alkyl, C₁-C₄-Alkoxy oder C₆-C₁₀-Aryl bedeuten.

Eine noch bevorzugtere Ausführungsform des erfindungsgemäßen Verfahrens ist dadurch gekennzeichnet, dass man einen Aldehyd der Formel (IIa), worin R'₁, R'₅, R₂ und R₄ Wasserstoff bedeuten, und X C₁-C₄-Alkyl, insbesondere Methyl ist, zu einem Aldehyd der Formel (la) umsetzt, worin R₁, R₂, R₄ und R₅ Wasserstoff bedeuten.

Eine weitere noch bevorzugtere Ausführungsform des erfindungsgemäßen Verfahrens ist dadurch gekennzeichnet, dass man einen Aldehyd der Formel (IIa), worin R'₁, R'₅ und R₄ Wasserstoff bedeutet und R₂ C₁-C₄-Alkoxy, insbesondere Methoxy, ist, und X C₁-C₄-Alkyl, insbesondere Methyl bedeutet, zu einem Aldehyd der Formel (la) umsetzt, worin R₁, R₄ und R₅ Wasserstoff bedeuten und R₂ C₁-C₄-Alkoxy, insbesondere Methoxy, ist.

Eine ebenfalls noch bevorzugtere Ausführungsform des erfindungsgemäßen Verfahrens ist dadurch gekennzeichnet, dass man einen Aldehyd der Formel (IIa), worin R'₁ und R'₅ Wasserstoff bedeutet und R₂ und R₄ C₁-C₄-Alkoxy, insbesondere Methoxy, ist, und X Methyl bedeutet, zu einem Aldehyd der Formel (la) umsetzt, worin R₁ und R₅ Wasserstoff bedeuten und R₂ und R₄ C₁-C₄-Alkoxy, insbesondere Methoxy, ist.

Eine weitere noch bevorzugtere Ausführungsform des erfindungsgemäßen Verfahrens ist dadurch gekennzeichnet, dass man einen Aldehyd der Formel (IIc) 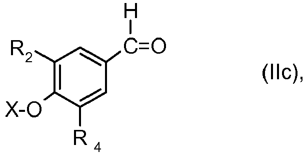 worin R₂ und R₄ unabhängig voneinander Wasserstoff oder C₁-C₄-Alkoxy, vorzugsweise Wasserstoff, Methoxy oder Ethoxy, ist und X C₁-C₄-Alkyl, vorzugsweise Methyl, bedeutet, zu einem Aldehyd der Formel (Ic) 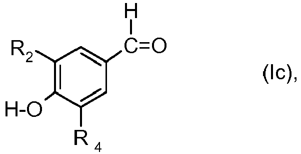 umsetzt, worin R₂ und R₄ die unter Formel (IIc) angegebenen Bedeutungen haben. Die Umsetzung erfolgt vorzugsweise in Gegenwart von Dimethylamin bei einer Temperatur im Bereich zwischen 130 und 150°C und einem Druck von 5 bis 15 bar. Nach einer Reaktionszeit von 12 bis 15 Stunden ist die Umsetzung beendet.

Als Ausgangsverbindungen für das erfindungsgemäße Verfahren kommen z.B. folgende Verbindungen der Formel (II) in Betracht: 2-Methoxybenzaldehyd, 4-Methoxybenzaldehyd, 2-Ethoxybenzaldehyd, 4-Ethoxybenzaldehyd, 3,4-Dimethoxybenzaldehyd, 3,4-Diethoxybenzaldehyd, 3,4,5-Trimethoxybenzaldehyd, 3-Methyl-4-methoxybenzaldehyd, 3-Ethyl-4-methoxybenzaldehyd, 2-Methyl-5-methoxybenzaldehyd, 2,5-Dimethyl-4-methoxybenzaldehyd, 2-Phenyl-4-ethoxybenzaldehyd.

Die erfindungsgemäße Umsetzung erfolgt unter Druck, so dass druckfeste Apparaturen eingesetzt werden müssen. Als druckfeste Apparate kommen handelsübliche Autoklaven oder auch kontinuierlich betriebene druckfeste Apparate, wie beispielsweise Rohrreaktoren in Betracht.

Autoklaven müssen höheren Drücken standhalten können. Übliche Laborautoklaven widerstehen ca. 150 Bar. Die Außenwände sind dickwandig und bestehen oft aus nichtrostenden Stählen, um Korrosionen zu vermeiden und das Beschickungsgut nicht zu verunreinigen. Für die Durchführung von Reaktionen mit aggressiven Chemikalien sind Autoklaven mit Innenbeschichtungen aus Teflon erhältlich. Spezielle Bauformen erlauben Drücke bis 7000 Bar und Temperaturen über 600 °C. Im Labor sind Autoklaven mit einem Volumen von wenigen Millilitern bis zu einigen Litern verbreitet, die üblicherweise über ein Manometer, ein Thermometer und ein Gasventil verfügen.

Die gemäß dem erfindungsgemäßen Verfahren erhaltenen Reaktionsprodukte werden durch Aufarbeitung des Reaktionsgemisches nach üblichen Verfahren wie z.B. Extraktion, Destillation und/oder Kristallisation gewonnen. Beispielsweise werden überschüssiges Dimethylamin und Wasser unter vermindertem Druck entfernt. Anschliessend wird ein mit Wasser nicht mischbares Lösungsmittel zugegeben, wie z.B. Diethylether, Methyltertbutylether oder ähnliche, die Mischung wird mit einer Säure, wie z.B. 30%-iger HCl in Wasser, sauer gestellt und nach der Phasentrennung und nach Entfernen aller flüchtigen Bestandteile der organischen Phase kann das Produkt in guter Ausbeute gegebenenfalls nach Destillation oder Kristallisation erhalten werden.

Die erfindungsgemäß durchzuführende Umsetzung kann wahlweise diskontinuierlich oder kontinuierlich durchgeführt werden. Dabei kann man beispielsweise im diskontinuierlichen Fall die Umsetzung so vornehmen, dass man den Aldehyd der Formel (II) und z.B. eine wässrige Lösung des Dialkylamins gegebenenfalls in Gegenwart eines weiteren Lösungsmittel wie z.B. Methanol in einem geeigneten Reaktionsgefäß, wie z.B. einem Autoklaven vorlegt und die Umsetzung bei erhöhter Temperatur und unter erhöhtem Druck vornimmt. Nach Abschluss der Reaktion werden, wie weiter oben beschrieben überschüssiges Dialkylamin und Wasser unter vermindertem Druck entfernt, ein nicht wasserlösliches Lösungsmittel, wie z.B. Methyltertbutylether, zugesetzt, die Mischung mit z.B. 30%-iger HCl in Wasser sauer gestellt und nach Phasentrennung und Entfernung aller flüchtigen Bestandteile der organischen Phase das Reaktionsprodukt der Formel (I) durch geeignete Trennverfahren vom erhaltenen Reaktionsgemisch isoliert. Die Reihenfolge des Inkontaktbringens der einzelnen Reaktionskomponenten ist nicht kritisch und kann nach Maßgabe der jeweiligen verfahrenstechnischen Ausgestaltung variiert werden.

Im Rahmen einer bevorzugten Ausführungsform führt man die erfindungsgemäß durchzuführende Dealkylierung, insbesondere die Demethylierung, des Aldehyds der Formel (II) in Gegenwart des Dialkylamins kontinuierlich durch, beispielsweise in einem kontinuierlich betriebenen Rohrreaktor bei erhöhter Temperatur und erhöhtem Druck. Dazu kann beispielsweise eine Mischung der umzusetzenden Ausgangsstoffe, der Aldehyd der Formel (II) und das Dialkylamin gegebenenfalls als wässrige Lösung, zubereitet werden und diese Mischung kontinuierlich miteinander in Kontakt gebracht werden. Dazu können die gewählten Ausgangskomponenten, Aldehyd der Formel (II) und Dialkylamin in einen Rohrreaktor eingebracht werden und die Ausgangsstoffe in diesen kontinuierlich eingetragen werden und das Reaktionsgemisch kontinuierlich ausgetragen werden.

Die Erfindung wird durch die folgenden Beispiele näher veranschaulicht ohne sie darauf zu beschränken. In den Beispielen verstehen sich alle Angaben in % als Gew.-%.

### Beispiel 1

In einen 100 ml Glasautoklav werden 8,84 g (65,0 mmol) 4-Methoxybenzaldehyd und 51,2 g (455 mmol) Dimethylamin (40 %ige Lösung in Wasser) gefüllt. Der Autoklav wird verschlossen und die zweiphasige Reaktionsmischung für 15 h bei 140 °C (Eigendruck 7,5 bar) gerührt. Überschüssiges Dimethylamin und Wasser werden unter vermindertem Druck entfernt, Methyltertbutylether zugegeben und die Mischung mit 30 %iger Salzsäure in Wasser sauer gestellt. Nach Phasentrennung und Entfernen aller flüchtigen Bestandteile der organischen Phase erhält man 6,5 g (0,53 mmol, 81 % Ausbeute) 4-Hydroxybenzaldehyd.

### Beispiel 2

In einen Metallautoklav werden 14,0 g (84,0 mmol) 3,4-Dimethoxybenzaldehyd und 120 g (1,0 mol) Dimethylamin (40 %ige Lösung in Wasser) gefüllt. Der Autoklav wird verschlossen und die zweiphasige Reaktionsmischung für 10 h bei 140 °C (Eigendruck 14 bar) gerührt. Überschüssiges Dimethylamin und Wasser werden unter vermindertem Druck entfernt, Methyltertbutylether zugegeben und die Mischung mit 30 %iger Salzsäure in Wasser sauer gestellt. Nach Phasentrennung und Entfernen aller flüchtigen Bestandteile der organischen Phase erhält man 8,5 g (56,0 mmol, 67 % Ausbeute) 4-Hydroxy-3-methoxybenzaldehyd.

### Beispiel 3

In einen 100 ml Glasautoklav werden 11,8 g (60,0 mmol) 3,4,5-Trimethoxybenzaldehyd und 47,2 g (420 mmol) Dimethylamin (40 %ige Lösung in Wasser) gefüllt. Der Autoklav wird verschlossen und die zweiphasige Reaktionsmischung für 18 h bei 140 °C (Eigendruck 10 bar) gerührt. Überschüssiges Dimethylamin und Wasser werden unter vermindertem Druck entfernt, Methyltertbutylether zugegeben und die Mischung mit 30 %iger Salzsäure in Wasser sauer gestellt. Nach Phasentrennung und Entfernen aller flüchtigen Bestandteile der organischen Phase erhält man 4-Hydroxy-3,5-dimethoxybenzaldehyd in einer Ausbeute von 72 %.

## Patentansprüche

1. Verfahren zur Herstellung eines Aldehyds der Formel (I) 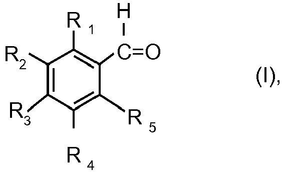 wobei einer, zwei oder alle drei Reste der Gruppe R₁, R₃ und R₅ Hydroxy bedeuten, und derjenige Rest oder diejenigen Reste der Gruppe R₁, R₃ und R₅ die nicht Hydroxy bedeuten, unabhängig voneinander Wasserstoff, C₁-C₈-Alkyl oder C₆-C₁₄-Aryl bedeuten, und R₂ und R₄ unabhängig voneinander Wasserstoff, C₁-C₈-Alkyl, wobei die genannten C1-C8-Alkylreste substituiert sein können, C₁-C₈-Alkoxy oder C₆-C₁₄-Aryl bedeuten, **dadurch gekennzeichnet, dass** man einen Aldehyd der Formel (II) 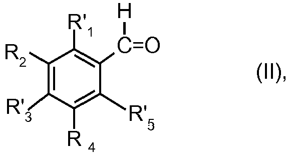 worin einer, zwei oder alle drei Reste der Gruppe R'₁, R'₃ und R'₅ C₁-C₈-Alkoxy bedeuten, und derjenige Rest oder diejenigen Reste der Gruppe R'₁, R'₃ und R'₅ die nicht C₁-C₈-Alkoxy bedeuten, unabhängig voneinander Wasserstoff, C₁-C₈-Alkyl oder C₆-C₁₄-Aryl bedeuten, und R₂ und R₄ die unter Formel (I) angegebenen Bedeutungen haben, bei einer Temperatur im Bereich zwischen 40 und 300°C und unter Druck zwischen 1 und 100 bar in Gegenwart von Dimethylamin oder Diethylamin umsetzt, und anschliessend das Reaktionsprodukt der Formel (I) isoliert.

2. Verfahren gemäß Anspruch 1, **dadurch gekennzeichnet, dass** die Umsetzung bei einer Temperatur zwischen 60 und 250°C erfolgt.

3. Verfahren gemäß einem der Ansprüche 1 bis 2, **dadurch gekennzeichnet, dass** die Umsetzung bei einem Druck zwischen 2 und 60 bar erfolgt.

4. Verfahren gemäß einem der Ansprüche 1 bis 3, **dadurch gekennzeichnet, dass** die Reaktionsdauer zwischen 1 und 48 Stunden, vorzugsweise zwischen 2 und 18 Stunden, beträgt.

5. Verfahren gemäß einem der Ansprüche 1 bis 4, **dadurch gekennzeichnet, dass** die Umsetzung in Gegenwart von Dimethylamin oder Diethylamin in wässriger Lösung erfolgt.

6. Verfahren gemäß Anspruch 5, **dadurch gekennzeichnet, dass** die Umsetzung in Gegenwart von Dimethylamin in wässriger Lösung erfolgt.

7. Verfahren gemäß einem der Ansprüche 1 bis 6, **dadurch gekennzeichnet, dass** man einen Aldehyd der Formel (II) einsetzt, worin einer, zwei oder alle drei Reste der Gruppe R'₁, R'₃ und R'₅ C₁-C₄-Alkoxy bedeuten, und derjenige Rest oder diejenigen Reste der Gruppe R'₁, R'₃ und R'₅ die nicht C₁-C₄-Alkoxy bedeuten, unabhängig voneinander Wasserstoff, C₁-C₄-Alkyl oder C₆-C₁₀-Aryl bedeuten, und R₂ und R₄ die unter Formel (I) in Anspruch 1 angegebenen Bedeutungen haben.

8. Verfahren gemäß einem der Ansprüche 1 bis 7, **dadurch gekennzeichnet, dass** man einen Aldehyd der Formel (II) einsetzt, worin einer, zwei oder alle drei Resteder Gruppe R'₁, R'₃ und R'₅ C₁-C₄-Alkoxy, vorzugsweise Methoxy, bedeuten, und derjenige Rest oder diejenigen Reste der Gruppe R'₁, R'₃ und R'₅ die nicht C₁-C₄-Alkoxy, vorzugsweise Methoxy, bedeuten, unabhängig voneinander Wasserstoff, C₁-C₄-Alkyl oder C₆-C₁₀-Aryl bedeuten, und R₂ und R₄ Wasserstoff, C₁-C₄-Alkyl, C₁-C₄-Alkoxy oder C₆-C₁₀-Aryl bedeuten.

9. Verfahren gemäß Anspruch 8, **dadurch gekennzeichnet, dass** man einen Aldehyd der Formel (IIa), 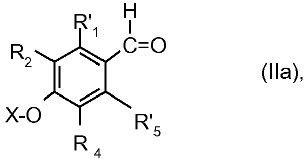 worin R'₁ und R'₅ unabhängig voneinander Wasserstoff, C₁-C₄-Alkyl oder C₆-C₁₀-Aryl bedeuten, und R₂ und R₄ unabhängig voneinander Wasserstoff, C₁-C₄-Alkyl, C₁-C₄-Alkoxy oder C₆-C₁₀-Aryl bedeuten, und X C₁-C₄-Alkyl, insbesondere Methyl ist, zu einem Aldehyd der Formel (la) 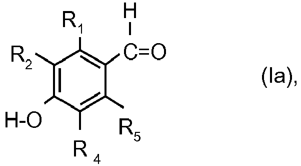 umsetzt, worin R₁ und R₅ unabhängig voneinander Wasserstoff, C₁-C₄-Alkyl oder C₆-C₁₀-Aryl bedeuten, und R₂ und R₄ unabhängig voneinander Wasserstoff, C₁-C₄-Alkyl, C₁-C₄-Alkoxy oder C₆-C₁₀-Aryl bedeuten.

10. Verfahren gemäß Anspruch 8, **dadurch gekennzeichnet, dass** man einen Aldehyd der Formel (IIb), 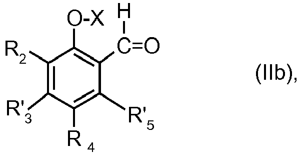 worin R'₃ und R'₅ unabhängig voneinander Wasserstoff, C₁-C₄-Alkyl oder C₆-C₁₀-Aryl bedeuten, und R₂ und R₄ unabhängig voneinander Wasserstoff, C₁-C₄-Alkyl, C₁-C₄-Alkoxy oder C₆-C₁₀-Aryl bedeuten, und X C₁-C₄-Alkyl, insbesondere Methyl ist, zu einem Aldehyd der Formel (Ib) 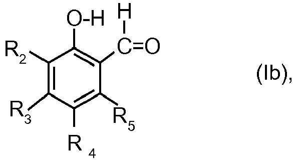 umsetzt, worin R₃ und R₅ unabhängig voneinander Wasserstoff, C₁-C₄-Alkyl oder C₆-C₁₀-Aryl bedeuten, und R₂ und R₄ unabhängig voneinander Wasserstoff, C₁-C₄-Alkyl, C₁-C₄-Alkoxy oder C₆-C₁₀-Aryl bedeuten.

11. Verfahren gemäß Anspruch 9, **dadurch gekennzeichnet, dass** man einen Aldehyd der Formel (IIa), worin R'₁, R'₅, R₂ und R₄ Wasserstoff bedeuten, und X C₁-C₄-Alkyl, insbesondere Methyl ist, zu einem Aldehyd der Formel (la) umsetzt, worin R₁, R₂, R₄ und R₅ Wasserstoff bedeuten.

12. Verfahren gemäß Anspruch 9, **dadurch gekennzeichnet, dass** man einen Aldehyd der Formel (IIa), worin R'₁, R'₅ und R₄ Wasserstoff bedeutet und R₂ C₁-C₄-Alkoxy, insbesondere Methoxy, ist, und X C₁-C₄-Alkyl, insbesondere Methyl, bedeutet, zu einem Aldehyd der Formel (la) umsetzt, worin R₁, R₄ und R₅ Wasserstoff bedeuten und R₂ C₁-C₄-Alkoxy, insbesondere Methoxy, ist.

13. Verfahren gemäß Anspruch 9, **dadurch gekennzeichnet, dass** man einen Aldehyd der Formel (IIa), worin R'₁, und R'₅ Wasserstoff bedeutet und R₂ und R₄ C₁-C₄-Alkoxy, insbesondere Methoxy, ist, und X Methyl bedeutet, zu einem Aldehyd der Formel (la) umsetzt, worin R₁ und R₅ Wasserstoff bedeuten und R₂ und R₄ C₁-C₄-Alkoxy, insbesondere Methoxy, ist.

14. Verfahren gemäß Anspruch 9, **dadurch gekennzeichnet, dass** man einen Aldehyd der Formel (IIc) 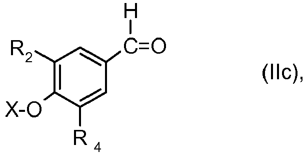 worin R₂ und R₄ unabhängig voneinander Wasserstoff oder C₁-C₄-Alkoxy, vorzugsweise Wasserstoff, Methoxy oder Ethoxy, ist und X C₁-C₄-Alkyl, vorzugsweise Methyl, bedeutet, zu einem Aldehyd der Formel (Ic) 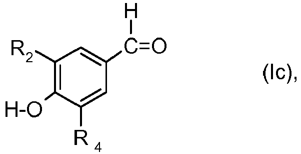 umsetzt, worin R₂ und R₄ die unter Formel (IIc) angegebenen Bedeutungen haben

15. Verfahren gemäß einem der Ansprüche 1 bis 14, **dadurch gekennzeichnet, dass** die Umsetzung kontinuierlich erfolgt.

## Claims

1. A process for preparing an aldehyde of the formula (I) 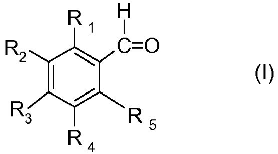 where one, two or all three radicals from the group of R₁, R₃ and R₅ are hydroxyl, and that radical or those radicals from the group of R₁, R₃ and R₅ which are not hydroxyl are each independently hydrogen, C₁-C₈-alkyl or C₆-C₁₄-aryl, and R₂ and R₄ are each independently hydrogen, C₁-C₈-alkyl, where said C₁-C₈ alkyl radicals may be substituted, C₁-C₈-alkoxy or C₆-C₁₄-aryl, which comprises converting an aldehyde of the formula (II) 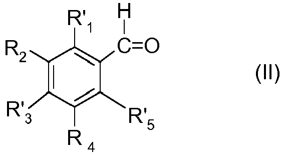 in which one, two or all three radicals from the group of R'₁, R'₃ and R'₅ are C₁-C₈-alkoxy, and that radical or those radicals from the group of R'₁, R'₃ and R'₅ which are not C₁-C₈-alkoxy are each independently hydrogen, C₁-C₈-alkyl or C₆-C₁₄-aryl, and R₂ and R₄ are each as defined for formula (I), at a temperature in the range between 40 and 300°C and under a pressure between 1 and 100 bar in the presence of dimethylamine or diethylamine, and then isolating the reaction product of the formula (I).

2. The process according to claim 1, wherein the conversion is effected at a temperature between 60 and 250°C.

3. The process according to any of claims 1 to 2, wherein the conversion is effected at a pressure between 2 and 60 bar.

4. The process according to any of claims 1 to 3, wherein the reaction time is between 1 and 48 hours, preferably between 2 and 18 hours.

5. The process according to any of claims 1 to 4, wherein the conversion is effected in the presence of dimethylamine or diethylamine in aqueous solution.

6. The process according to claim 5, wherein the conversion is effected in the presence of dimethylamine in aqueous solution.

7. The process according to any of claims 1 to 6, wherein an aldehyde of the formula (II) in which one, two or all three radicals from the group of R'₁, R'₃ and R'₅ are C₁-C₄-alkoxy, and that radical or those radicals from the group of R'₁, R'₃ and R'₅ which are not C₁-C₄-alkoxy are each independently hydrogen, C₁-C₄-alkyl or C₆-C₁₀-aryl, and R₂ and R₄ are each as defined for formula (I) in claim 1, is used.

8. The process according to any of claims 1 to 7, wherein an aldehyde of the formula (II) in which one, two or all three radicals from the group of R'₁, R'₃ and R'₅ are C₁-C₄ -alkoxy, preferably methoxy, and that radical or those radicals from the group of R'₁, R'₃ and R'₅ which are not C₁-C₄-alkoxy, preferably methoxy, are each independently hydrogen, C₁-C₄-alkyl or C₆-C₁₀-aryl, and R₂ and R₄ are each hydrogen, C₁-C₄-alkyl, C₁-C₄-alkoxy or C₆-C₁₀-aryl, is used.

9. The process according to claim 8, wherein an aldehyde of the formula (IIa) 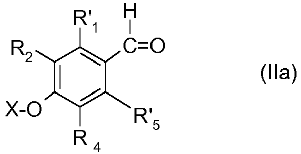 in which R'₁ and R'₅ are each independently hydrogen, C₁-C₄-alkyl or C₆-C₁₀-aryl, and R₂ and R₄ are each independently hydrogen, C₁-C₄-alkyl, C₁-C₄-alkoxy or C₆-C₁₀-aryl, and X is C₁-C₄-alkyl, especially methyl, is converted to an aldehyde of the formula (Ia) 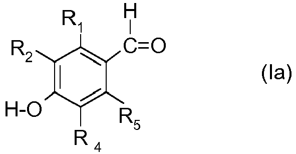 in which R₁ and R₅ are each independently hydrogen, C₁-C₄-alkyl or C₆-C₁₀-aryl, and R₂ and R₄ are each independently hydrogen, C₁-C₄-alkyl, C₁-C₄-alkoxy or C₆-C₁₀-aryl.

10. The process according to claim 8, wherein an aldehyde of the formula (IIb), 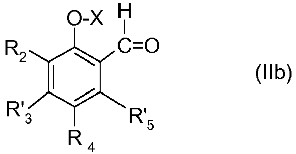 in which R'₃ and R'₅ are each independently hydrogen, C₁-C₄-alkyl or C₆-C₁₀-aryl, and R₂ and R₄ are each independently hydrogen, C₁-C₄-alkyl, C₁-C₄-alkoxy or C₆-C₁₀-aryl, and X is C₁-C₄-alkyl, especially methyl, is converted to an aldehyde of the formula (Ib) 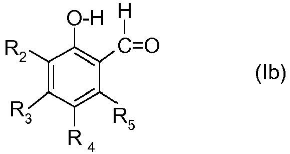 in which R₃ and R₅ are each independently hydrogen, C₁-C₄-alkyl or C₆-C₁₀-aryl, and R₂ and R₄ are each independently hydrogen, C₁-C₄-alkyl, C₁-C₄-alkoxy or C₆-C₁₀-aryl.

11. The process according to claim 9, wherein an aldehyde of the formula (IIa) in which R'₁, R'5, R2 and R4 are each hydrogen, and X is C1-C4-alkyl, especially methyl, is converted to an aldehyde of the formula (Ia) in which R₁, R2, R4 and R5 are each hydrogen.

12. The process according to claim 9, wherein an aldehyde of the formula (IIa) in which R'₁, R'₅ and R4 are each hydrogen and R2 is C1-C4-alkoxy, especially methoxy, and X is C1-C4-alkyl, especially methyl, is converted to an aldehyde of the formula (Ia) in which R1, R4 and R5 are each hydrogen and R2 is C1-C4-alkoxy, especially methoxy.

13. The process according to claim 9, wherein an aldehyde of the formula (IIa) in which R'₁ and R'₅ are each hydrogen and R₂ and R₄ are each C₁-C₄-alkoxy, especially methoxy, and X is methyl, is converted to an aldehyde of the formula (Ia) in which R₁ and R₅ are each hydrogen and R₂ and R₄ are each C₁-C₄-alkoxy, especially methoxy.

14. The process according to claim 9, wherein an aldehyde of the formula (IIc) 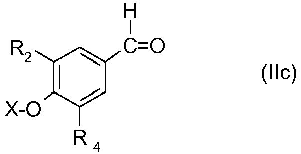 in which R₂ and R₄ are each independently hydrogen or C₁-C₄-alkoxy, preferably hydrogen, methoxy or ethoxy, and X is C₁-C₄-alkyl, preferably methyl, is converted to an aldehyde of the formula (Ic) 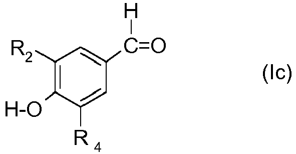 in which R₂ and R₄ are each as defined for formula (IIc).

15. The process according to any of claims 1 to 14, wherein the conversion is effected continuously.

## Revendications

1. Procédé de fabrication d'un aldéhyde de formule (I) 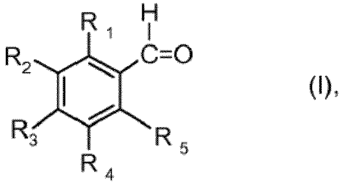 dans laquelle un, deux ou les trois radicaux des groupes R₁, R₃ et R₅ signifient hydroxy, et le radical ou les radicaux des groupes R₁, R₃ et R₅ qui ne signifient pas hydroxy signifient indépendamment l'un de l'autre hydrogène, alkyle en C₁-C₈ ou aryle en C₆-C₁₄, et R₂ et R₄ signifient indépendamment l'un de l'autre hydrogène, alkyle en C₁-C₈, les radicaux alkyle en C₁-C₈ indiqués pouvant être substitués, alcoxy en C₁-C₈ ou aryle en C₆-C₁₄, **caractérisé en ce qu'**un aldéhyde de formule (II) 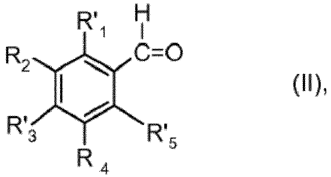 dans laquelle un, deux ou les trois radicaux des groupes R'₁, R'₃ et R'₅ signifient alcoxy en C₁-C₈, et le radical ou les radicaux des groupes R'₁, R'₃ et R'₅ qui ne signifient pas alcoxy en C₁-C₈ signifient indépendamment l'un de l'autre hydrogène, alkyle en C₁-C₈ ou aryle en C₆-C₁₄, et R₂ et R₄ ont les significations indiquées pour la formule (I), est mis en réaction à une température dans la plage comprise entre 40 et 300 °C et à une pression comprise entre 1 et 100 bar en présence de diméthylamine ou de diéthylamine, puis le produit de réaction de formule (I) est isolé.

2. Procédé selon la revendication 1, **caractérisé en ce que** la réaction a lieu à une température comprise entre 60 et 250 °C.

3. Procédé selon l'une quelconque des revendications 1 à 2, **caractérisé en ce que** la réaction a lieu à une pression comprise entre 2 et 60 bar.

4. Procédé selon l'une quelconque des revendications 1 à 3, **caractérisé en ce que** la durée de la réaction est comprise entre 1 et 48 heures, de préférence entre 2 et 18 heures.

5. Procédé selon l'une quelconque des revendications 1 à 4, **caractérisé en ce que** la réaction a lieu en présence de diméthylamine ou de diéthylamine en solution aqueuse.

6. Procédé selon la revendication 5, **caractérisé en ce que** la réaction a lieu en présence de diméthylamine en solution aqueuse.

7. Procédé selon l'une quelconque des revendications 1 à 6, **caractérisé en ce qu'**un aldéhyde de formule (II) est utilisé, dans lequel un, deux ou les trois radicaux des groupes R'₁, R'₃ et R'₅ signifient alcoxy en C₁-C₄, et le radical ou les radicaux des groupes R'₁, R'₃ et R'₅ qui ne signifient pas alcoxy en C₁-C₄ signifient indépendamment l'un de l'autre hydrogène, alkyle en C₁-C₄ ou aryle en C₆-C₁₀, et R₂ et R₄ ont les significations indiquées pour la formule (I) dans la revendication 1.

8. Procédé selon l'une quelconque des revendications 1 à 7, **caractérisé en ce qu'**un aldéhyde de formule (II) est utilisé, dans lequel un, deux ou les trois radicaux des groupes R'₁, R'₃ et R'₅ signifient alcoxy en C₁-C₄, de préférence méthoxy, et le radical ou les radicaux des groupes R'₁, R'₃ et R'₅ qui ne signifient pas alcoxy en C₁-C₄, de préférence méthoxy, signifient indépendamment l'un de l'autre hydrogène, alkyle en C₁-C₄ ou aryle en C₆-C₁₀, et R₂ et R₄ signifient hydrogène, alkyle en C₁-C₄, alcoxy en C₁-C₄ ou aryle en C₆-C₁₀.

9. Procédé selon la revendication 8, **caractérisé en ce qu'**un aldéhyde de formule (IIa) 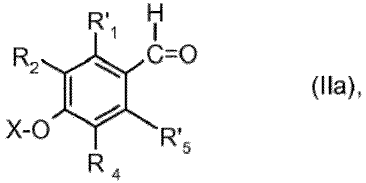 dans laquelle R'₁ et R'₅ signifient indépendamment l'un de l'autre hydrogène, alkyle en C₁-C₄ ou aryle en C₆-C₁₀, et R₂ et R₄ signifient indépendamment l'un de l'autre hydrogène, alkyle en C₁-C₄, alcoxy en C₁-C₄ ou aryle en C₆-C₁₀, et X signifie alkyle en C₁-C₄, notamment méthyle, est transformé en un aldéhyde de formule (Ia) 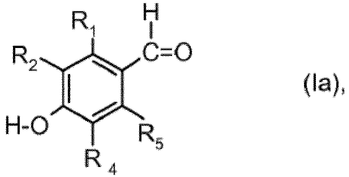 dans laquelle R₁ et R₅ signifient indépendamment l'un de l'autre hydrogène, alkyle en C₁-C₄ ou aryle en C₆-C₁₀, et R₂ et R₄ signifient indépendamment l'un de l'autre hydrogène, alkyle en C₁-C₄, alcoxy en C₁-C₄ ou aryle en C₆-C₁₀.

10. Procédé selon la revendication 8, **caractérisé en ce qu'**un aldéhyde de formule (IIb) 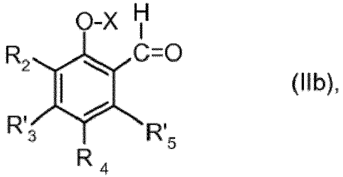 dans laquelle R'₃ et R'₅ signifient indépendamment l'un de l'autre hydrogène, alkyle en C₁-C₄ ou aryle en C₆-C₁₀, et R₂ et R₄ signifient indépendamment l'un de l'autre hydrogène, alkyle en C₁-C₄, alcoxy en C₁-C₄ ou aryle en C₆-C₁₀, et X signifie alkyle en C₁-C₄, notamment méthyle, est transformé en un aldéhyde de formule (Ib) 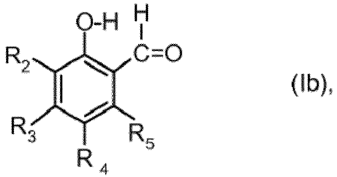 dans laquelle R₃ et R₅ signifient indépendamment l'un de l'autre hydrogène, alkyle en C₁-C₄ ou aryle en C₆-C₁₀, et R₂ et R₄ signifient indépendamment l'un de l'autre hydrogène, alkyle en C₁-C₄, alcoxy en C₁-C₄ ou aryle en C₆-C₁₀.

11. Procédé selon la revendication 9, **caractérisé en ce qu'**un aldéhyde de formule (IIa), dans laquelle R'₁, R'₅, R₂ et R₄ signifient hydrogène, et X signifie alkyle en C₁-C₄, notamment méthyle, est transformé en un aldéhyde de formule (Ia), dans laquelle R₁, R₂, R₄ et R₅ signifient hydrogène.

12. Procédé selon la revendication 9, **caractérisé en ce qu'**un aldéhyde de formule (IIa), dans laquelle R'₁, R'₅ et R₄ signifient hydrogène, et R₂ signifie alcoxy en C₁-C₄, notamment méthoxy, et X signifie alkyle en C₁-C₄, notamment méthyle, est transformé en un aldéhyde de formule (Ia), dans laquelle R₁, R₄ et R₅ signifient hydrogène et R₂ signifie alcoxy en C₁-C₄, notamment méthoxy.

13. Procédé selon la revendication 9, **caractérisé en ce qu'**un aldéhyde de formule (IIa), dans laquelle R'₁ et R'₅ signifient hydrogène, et R₂ et R₄ signifient alcoxy en C₁-C₄, notamment méthoxy, et X signifie méthyle, est transformé en un aldéhyde de formule (Ia), dans laquelle R₁ et R₅ signifient hydrogène et R₂ et R₄ signifient alcoxy en C₁-C₄, notamment méthoxy.

14. Procédé selon la revendication 9, **caractérisé en ce qu'**un aldéhyde de formule (IIc) 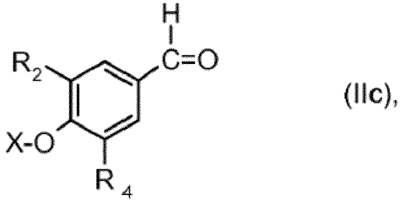 dans laquelle R₂ et R₄ signifient indépendamment l'un de l'autre hydrogène ou alcoxy en C₁-C₄, de préférence hydrogène, méthoxy ou éthoxy, et X signifie alkyle en C₁-C₄, de préférence méthyle, est transformé en un aldéhyde de formule (Ic) 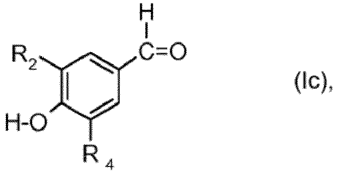 dans laquelle R₂ et R₄ ont les significations indiquées pour la formule (IIc).

15. Procédé selon l'une quelconque des revendications 1 à 14, **caractérisé en ce que** la réaction a lieu en continu.
